# EUROPÄISCHE PATENTSCHRIFT

(11) **EP 1 355 575 B1**
(45) Veröffentlichungstag und Bekanntmachung des Hinweises auf die Patenterteilung: **09.06.2004**
(21) Anmeldenummer: 01985804.2
(22) Anmeldetag: 22.12.2001
(51) Int. Cl.: A61B 17/068

(54) **VORRICHTUNG ZUM EINBRINGEN UND FORMEN VON HAUTKLAMMERN**
DEVICE FOR THE INTRODUCTION AND FASHIONING OF SKIN CLIPS
DISPOSITIF D'APPLICATION ET DE MODELAGE D'AGRAFES CHIRURGICALES

(30) Priorität: 31.01.2001 DE 10104532
(43) Veröffentlichungstag der Anmeldung: 29.10.2003
(73) Patentinhaber: Eberhard-Karls-Universität Tübingen, 72076 Tübingen (DE)
(72) Erfinder: BREUNINGER, Helmut, 72076 Tübingen (DE)
(74) Vertreter: Roth, Klaus, Dr.
(86) Internationale Anmeldenummer: PCT/DE2001/004876
(87) Internationale Veröffentlichungsnummer: WO 2002/060327

(56) Entgegenhaltungen:
- WO-A-00/02490
- US-A- 4 317 451
- US-A- 4 485 816
- US-A- 4 887 756
- US-A- 5 902 310

## Beschreibung

Die Erfindung betrifft eine Vorrichtung zum Einbringen und Formen von Hautklammern nach dem Oberbegriff des Anspruchs 1. Eine solche Vorrichtung ist aus WO-A-0 002 490 bekannt.

Bislang werden zum Verbinden von Wundrändern Hautklammern verwendet, die zwei Seitenschenkel und ein die Seitenschenkel verbindendes Zwischenstück aufweisen. Die Seitenschenkel stehen hierbei rechtwinklig zum Zwischenstück und werden mit einem bekannten Klammerapparat beidseits der Wunde in die Haut eingetrieben, während die Wundränder durch geeignete Maßnahmen fixiert werden. Anschließend wird durch das Eindrücken eines u-förmigen Formstempels über einen auf der Wunde aufliegenden Gegenhalter die beiden Endbereiche des Zwischenstücks rechtwinklig umgebogen. Die beiden vorherigen Seitenschenkel der Hautklammer werden dabei ebenfalls umgebogen und kommen im Wesentlichen parallel zu dem mittleren, nicht umgebogenen Bereich des Zwischenstücks in der Haut zu liegen.

Dieser Formvorgang verursacht durch das extreme Verformen der Klammer in der Haut und dem damit verbundenen Eindrücken des Formstempels mit seinen beiden seitlichen Bereichen in die Oberfläche der Haut enorme Quetschungen des in diesem Bereich liegenden Gewebes. Diese Gewebequetschung führt zu einer ausgeprägten Narbenbildung während des anschließenden Heilungsprozesses. Weiterhin ist die Entfernung der solchermaßen gebogenen Hautklammern nur mit einem Spezialinstrument möglich, da die erheblich verformte Hautklammer zur Entfernung wieder verformt werden muss.

Die Erfindung hat demgegenüber die Aufgabe, eine Vorrichtung und ein Verfahren zum Einbringen und Formen von Hautklammern vorzuschlagen, bei der das Ausmaß der Gewebequetschung und deren negative Folgeerscheinungen vermindert und die Entfernung der Hautklammern nach dem Ausheilen der Wunde erleichtert wird.

Diese Aufgabe wird ausgehend von einer Vorrichtung der einleitend genannten Art durch die kennzeichnenden Merkmale des Anspruchs 1 gelöst.

Durch die in den Unteransprüchen genannten Maßnahmen sind vorteilhafte Ausführungen und Weiterbildungen der Erfindung möglich.

Dementsprechend zeichnet sich eine erfindungsgemäße Vorrichtung dadurch aus, dass im geformten Endzustand der Hautklammer die Abweichung der Seitenschenkel von der Einbringrichtung kleiner als 90° ist. Eine solche Formgebung lässt sich mit deutlich verringerten Gewebequetschungen und der Verminderung der oben angeführten nachteiligen Folgeerscheinungen durchführen. Weiterhin ist eine derart befestigte Hautklammer wesentlich leichter entfernbar.

In einer vorteilhaften Weiterbildung der Erfindung wird zur Herstellung dieser Seitenschenkelorientierung eine Verformung des Zwischenstücks vorgenommen. Die Verformung des Zwischenstücks ermöglicht die Bearbeitung der Hautklammer im Wesentlichen oberhalb der Haut, was wiederum der Verminderung von Gewebequetschungen dient.

In einer weiteren besonderen Ausführungsform der Erfindung wird wenigstens ein Gegenhalter vorgesehen. Ein solcher Gegenhalter, auf dem die Hautklammer während des Eindringens in die Haut anschlägt, ermöglicht die Aufnahme des Drucks, der für die Verformung der Klammer erforderlich ist, so dass die darunter befindliche Haut im Wesentlichen hiervon entlastet ist.

Vorzugsweise werden am Formstempel der Vorrichtung wenigstens zwei Formungszonen beidseits des Gegenhalters vorgesehen. Auf diese Weise ist eine Verformung des Zwischenstücks, nachdem es am Gegenhalter angeschlagen hat, durch Druckbeaufschlagung in Einbringrichtung der Hautklammer ohne Druckbelastung der darunter befindlichen Haut möglich. Bei dieser Verformung des Zwischenstücks werden zugleich die vorher parallel zur Eindringrichtung liegenden Seitenschenkel der Hautklammer in die gewünschte Lage gebracht.

Vorteilhafterweise wird der Gegenhalter, der den Druck des Formstempels bei der Verformung der Hautklammer aufnimmt, als Dorn ausgebildet. Ein solcher Dorn ermöglicht es, das Zwischenstück der Hautklammer bei der Verformung zu knicken. Das Knicken des länglichen Zwischenstücks der Hautklammer, außerhalb der Haut ist mit wesentlich weniger Verformungsdruck zu bewerkstelligen, als eine Verformung, die teilweise im Innern der Haut stattfindet. Diese Maßnahme dient demnach wiederum einer Verminderung von Hautquetschungen.

Vorzugsweise wird der Formstempel V-förmig ausgebildet. Durch diese V-Form wird ein zentraler Knick in der Endform der Hautklammer vorgegeben, wobei der Weg des Formstempels dadurch begrenzt ist, dass der Scheitelpunkt des V-Profils auf dem Gegenhalter anschlägt, der vorzugsweise wie oben angeführt, als Dorn ausgebildet wird. Grundsätzlich kann jedoch ein solcher Gegenhalter auch anderweitig ausgebildet, z.B. an das Profil des Formstempels angepasst werden. Im Falle eines V-förmigen Stempels könnte demnach auch der Gegenhalter ein komplementäres V-Profil aufweisen.

Die genannten Ausführungsbeispiele, die einen zentralen Knick im Zwischenstück der Hautklammer bevorzugen, sind nicht einschränkend zu verstehen. Grundsätzlich wäre es auch möglich, das Zwischenstück mit einem kontinuierlichen Profil derart zu verformen, dass die Seitenschenkel der Hautklammer die gewünschte Lage einnehmen. Ebenso wäre es möglich, das Zwischenstück mehrfach zu knicken, so dass sich der Verlauf eines Polygonzugs ergibt.

In der bevorzugten Ausführungsform, die mit vergleichsweise wenig Aufwand zu realisieren ist, wird jedoch ein im Ausgangszustand gerades und im geformten Endzustand geknicktes Zwischenstück der Hautklammer vorgesehen. Der zentrale Knick ist mit einem Minimum an Verformungsdruck zu bewerkstelligen, indem die druckbeaufschlagenden Zonen des Formstempels sich bis in die Randbereiche des Zwischenstücks der Hautklammer erstrecken, so dass sich eine Hebelwirkung auf das Zwischenstück der Hautklammer ergibt.

Vorteilhafterweise wird zusätzlich ein Magazin für mehrere Hautklammern im Ausgangszustand vorgesehen, wodurch eine Vielzahl von Hautklammern in einem Arbeitsgang nacheinander ohne "Nachladen" des Klammerapparats anzubringen sind, wie dies bei üblichen zu klammernden Wunden erforderlich ist.

Die Formgebung der Hautklammer wird dabei vorteilhafterweise so gewählt, dass die Abweichung der Seitenschenkel durch die Verformung oberhalb der Hautoberfläche von der Eindringrichtung in die Haut weniger als 45° beträgt. Grundsätzlich ist zur Erzielung der erfindungsgemäßen Vorteile eine Stellung der Seitenschenkel anzustreben, die möglichst nahe an den ursprünglichen Zustand mit parallel zur Eindringrichtung liegenden Seitenschenkeln herankommt. Dabei ist allerdings zu gewährleisten, dass die Heftfunktion der Klammer, d.h. das Zusammenhalten der Wundränder gewährleistet bleibt und die Hautklammer sich nicht selbsttätig aus der Haut lösen kann. Eine Abwinkelung der Seitenschenkel in einem Bereich von ungefähr 30° bietet dabei noch eine ausreichende Haltefunktion in Verbindung mit einer weitgehenden Vermeidung von Hautquetschungen. Zudem wird durch diese Stellung erreicht, dass sich die tieferen Anteile der Lederhaut fest aneinander legen, was für eine gute Wundheilung günstig ist.

Die Entfernung der Hautklammer ist ebenfalls umso leichter, je weniger die Seitenschenkel aus ihrer Einbringrichtung abgelenkt wurden. Bei der genannten Stellung von 30° gegenüber der Einbringrichtung lässt sich die Hautklammer mit herkömmlichen Instrumenten, beispielsweise eine Pinzette herausziehen, ohne dass eine Verformung der Hautklammer erforderlich ist. Die zwischen den beiden Seitenschenkeln liegende Haut stellt ein elastisches Gewebe dar und kann ihrerseits die für das Entfernen der Hautklammer erforderlichen Ausgleichsverformungen durchführen.

Der Aufwand zur Entfernung der Hautklammern wird weiterhin dadurch verringert, dass das Lösen der Hautklammer so einfach zu bewerkstelligen ist, dass es nicht notgedrungener Weise von einem Chirurgen oder entsprechend ausgebildetem Personal vorgenommen werden muss. Gegebenenfalls kann die Entfernung der Hautklammern sogar von den Patienten selbst bzw. von anderem nicht medizinisch ausgebildeten Personal vorgenommen werden. Dies wird zusätzlich dadurch unterstützt, dass hierfür keinerlei Spezialwerkzeug erforderlich ist, das wiederum den Gang zu einem entsprechendem Personal, z.B. einem Arzt erfordern würde.

Durch die Verringerung der Hautquetschungen wird die Narbenbildung deutlich reduziert. Dadurch wird diese Art der Wundversorgung einem breiteren Anwendungsgebiet zugänglich. Insbesondere dort wo sichtbare Wunden versorgt werden müssen, beispielsweise im Bereich der Unfall- oder Schönheitschirurgie kann auf diese Weise nunmehr eine Wundversorgung mit Hilfe einer Klammerung durchgeführt werden, wo bislang aus kosmetischen Gründen auf eine Klammerung verzichtet und eine erheblich aufwendigere Versorgung mit entsprechenden Nähten vorgenommen wurde.

Eine erfindungsgemäße Vorrichtung wird vorteilhafterweise so realisiert, dass sich bei einer leichten und einfachen Bauweise eine gute Reinigungsmöglichkeit ergibt. Darüber hinaus sollte die erfindungsgemäße Vorrichtung sterilisierbar und somit wiederverwendbar ausgebildet werden. Weiterhin sollte eine erfindungsgemäße Vorrichtung leicht, d.h. ohne Kraftaufwand handzuhaben sein, damit der Operateur seine Aufmerksamkeit nicht auf die Betätigung der Vorrichtung richten muss.

Diese Vorteile werden bei einer Vorrichtung erreicht, die ein Druckelement zum Eindrücken des Formstempels vorsieht, so dass die Kraft zum Anbringen der Hautklammer zumindest zum Zeitpunkt der Fixierung der Hautklammer nicht vom Operateur, sondern von dem Druckelement aufgebracht wird.

Ein solches Druckelement kann in einer besonders einfachen Ausführungsform als Druckfeder ausgebildet sein. Grundsätzlich können jedoch auch andere Druckelemente, z.B. Elektromotoren, pneumatische Zylinder oder dergleichen vorgesehen werden.

Weiterhin wird in einer bevorzugten Ausführungsform ein Rückholelement zur Rückholung des Formstempels vorgesehen, die im Falle eines Druckelementes wie oben angeführt, dem Druckelement entgegenwirkt. Ein solches Rückholelement kann in Form einer Hubfeder ausgebildet werden. Diese Ausführungsform bietet sich vor allem in Kombination mit einer Druckfeder als Druckelement an.

Um dabei die Kraft der Druckfeder zum Eindrücken des Formstempels nicht abzuschwächen, wird die Hubfeder vorteilhafterweise vom Formstempel abkoppelbar ausgebildet. Bei einer solchen Ausführungsform kann die Abkopplung mit Hilfe eines leicht zu betätigenden Auslöseelementes vorgenommen werden, wodurch das Anbringen der Hautklammer über die Druckfeder ohne Krafteinwirkung des Operateurs ausgelöst wird.

Zur Rückholung des Formstempels wird vorteilhafterweise ein Rastelement zum Verrasten der Hubfeder am Formstempel vorgesehen, so dass die Rückholung des Formstempels und somit gewissermaßen das Laden des Klammerapparates durch Spannen der Hubfeder von Seiten des Operateurs vorgenommen werden kann, wobei das Rastelement selbsttätig am Formstempel verrastet. Bemerkenswert ist hierbei, dass dieses Spannen der Hubfeder in nicht aufgesetztem Zustand auf der Haut des Patienten zu einem Zeitpunkt vorgenommen wird, in dem die Aufmerksamkeit des Operateurs nicht beeinträchtigt wird.

In einer besonders vorteilhaften Ausführungsform wird das Auslöseelement zugleich als Rastelement ausgebildet, wodurch sich der konstruktive Aufbau der Vorrichtung vereinfacht.

Weiterhin ist es von Vorteil, die Druck- und/oder die Hubfeder als Blattfeder auszubilden, mit denen sich ein griffiges Handgerät gestalten lässt. Insbesondere die Hubfeder ist hierbei der manuellen Einwirkung ohne weitere Betätigungselemente gut zugänglich.

In einer Weiterbildung der Erfindung wird zudem ein Stellelement zum Einstellen der Vorspannung der Druckund/oder der Hubfeder vorgesehen. Ein solches Verstellelement kann in einer einfachen Ausführungsform in Form einer Spannschraube vorgesehen werden. Die Einstellung der Druckkraft ist insbesondere dann von Vorteil, wenn unterschiedliche Klammergrößen und somit unterschiedliche Ausführungen von Formstempel mit ein und demselben Klammerapparat betätigt werden können. Für größere Hautklammern und entsprechend massivere Formstempel wird eine größere Vorspannung benötigt als für leichte Klammern mit entsprechend leichterem Formstempel.

In einer besonderen Ausführungsform der Erfindung wird zudem ein Schutzband vorgesehen, das zwischen Haut und Hautklammer eingelegt ist und mit dem Anbringen der Hautklammer auf der Wunde befestigt wird. Hierdurch werden die Wundränder abgedeckt, so dass die Wunde vor Verschmutzung, Infektionen, mechanische Einwirkungen, usw. geschützt ist.

In einer Weiterbildung dieser Ausführungsform wird ein Magazin, beispielsweise ein Stapel derartiger Schutzbänder oder eine Vorratsrolle vorgesehen, aus dem das Schutzband bei Bedarf entnehmbar ist. Eine erfindungsgemäße Vorrichtung kann ein solches Schutzband selbsttätig aus dem Vorratsmagazin entnehmen. Bei einer Vorratsrolle genügt es beispielsweise, wenn das Ende des Schutzbandes mit der ersten Hautklammer fixiert wird, so dass das Schutzband mit dem Versatz der gesamten Klammervorrichtung von der Rolle abgezogen und nach Beendigung des Klammervorgangs geschnitten wird.

Ein Ausführungsbeispiel der Erfindung ist in der Zeichnung dargestellt und wird anhand der Figuren nachfolgend näher erläutert.

Im Einzelnen zeigen
- Figur 1: eine schematische Darstellung einer erfindungsgemäßen Vorrichtung, die
- Figuren 2 bis 8: einzelne Behandlungsschritte bei der Wundversorgung mit einer erfindungsgemäßen Vorrichtung,
- Figur 9: eine schematische Schnittdarstellung einer erfindungsgemäßen Vorrichtung
- Figur 10: eine schematische Darstellung einer Vorderansicht einer erfindungsgemäßen Vorrichtung,
- Figur 11: eine schematische Skizze einer erfindungsgemäßen Klammervorrichtung vor dem Einbringen einer Hautklammer,
- Figur 12: eine Skizze gemäß Figur 11 nach der Auslösung eines Klammervorgangs und
- Figur 13: eine Skizze gemäß den Figuren 11 und 12 bei eingebrachter Hautklammer vor der Rückholung des Formstempels,
- Figur 14: eine schematische Darstellung einer erfindungsgemäßen Vorrichtung mit Schutzband in der Frontansicht,
- Figur 15: eine Darstellung einer versorgten Wunde mit eingelegtem Schutzband und

- Figur 16: eine schematische Seitenansicht der erfindungsgemäßen Vorrichtung mit Schutzband.

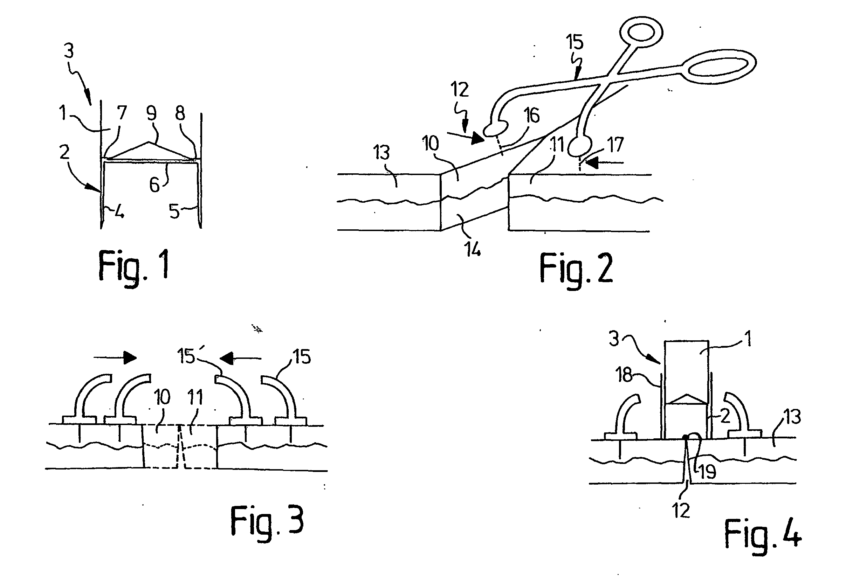

Figur 1 zeigt einen Formstempel 1 und eine darunter angeordnete Hautklammer 2, die zu einer erfindungsgemäßen Vorrichtung 3 gehören. Die Hautklammer 2 umfasst zwei Seitenschenkel 4, 5 mit spitzen Enden, die über ein Zwischenstück 6 miteinander verbunden sind. Der Formstempel 1 entspricht mit seiner Breite ungefähr der Breite der Hautklammer 2 bzw. des Zwischenstücks 6.

In den beiden äußeren Bereichen umfasst der Formstempel 1 jeweils eine druckbeaufschlagende Formzone 7, 8. Dazwischen ist der Formstempel 1 mit einer V-Kontur 9 versehen. Auf die Funktion der V-Kontur 9 wird weiter unten näher eingegangen.

Figur 2 zeigt die Wundränder 10, 11 einer Wunde 12. Hierbei ist die Lederhaut 13 mit darunter liegendem Fettgewebe 14 schematisch dargestellt, die im Bereich der Wunde 12 auseinanderklaffen.

Eine Adaptionszange 15, die mit zwei Spitzen 16, 17 in die Haut eindringt, dient dazu, die Wundränder aneinanderzufügen. Dieser Zustand ist in Figur 3 dargestellt. Die geöffnete Stellung der Adaptionszange 15 ist hierbei durchgehend schwarz, die geschlossene Stellung mit aneinandergefügten Wundrändern 10, 11 der Adaptionszange 15' ist schraffiert dargestellt.

Nachdem die Wundränder auf diese Weise zusammengeführt wurden, kommt eine erfindungsgemäße Vorrichtung 3 zum Einsatz, wie in Figur 4 dargestellt ist. Die Vorrichtung 3 umfasst neben der Hautklammer 2 und dem Formstempel 1 ein Führungsgehäuse 18 für den Formstempel 1 sowie einen Dorn 19, der als Gegenhalter für den Formstempel 1 dient. Im Verfahrensschritt gemäß Figur 4 ist die Vorrichtung 3 gerade auf die Lederhaut 13 so aufgesetzt, dass sie die Wunde 12 überdeckt.

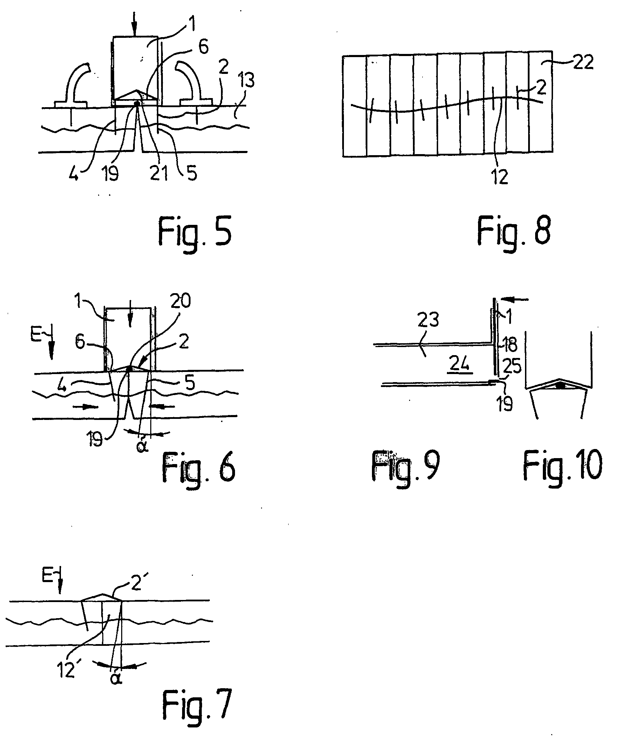

Im nächsten Verfahrensschritt (vgl. Figur 5) wird der Formstempel 1 niedergedrückt. Dieses Niederdrücken kann im Fall A durch eine Feder impulsartig, wie bei handlesüblichen "Takkerwerkzeugen" geschehen, um eine Penetration der senkrechten Klammerschenkel 4, 5 in die Lederhaut 13 und Fettgewebe 14 zu erzielen, ohne dass dieses ausweichen kann bis das Zwischenstück 6 der Hautklammer 2 auf dem Dorn 19 anstößt und im gleichen Vorgang der Formstempel 1 das Zwischenstück 6 der Hautklammer 2 derart verbiegt, dass sich oberhalb des Dornes 19 ein Knick 20 ausbildet.

Denkbar ist im Fall B auch ein Einbringen der senkrechten Seitenschenkel 4, 5 in die Lederhaut 13 bis zum Fettgewebe 14 indem zunächst die Klammer bis zum Anschlag des Dornes noch ohne Verformung derselben aus dem Magazin herausgedrückt wird und danach die Seitenschenkel 4, 5 ruckartig vom Benutzer niedergedrückt werden bis das Gerät auf der Haut aufliegt. Eventuell ist aber im Fall C bei spitz gestalteten Klammerenden auch die Einbringung ohne Impuls möglich.

In den beiden oben beschriebenen Fällen B und C wird im nächsten Verfahrensschritt der Formstempel 1 weiter eingedrückt, wie in Figur 6 veranschaulicht wird. Hierbei wird das Zwischenstück 6 der Hautklammer 2 derart verbogen, dass sich oberhalb des Dorns 19 ein Knick 20 ausbildet. Durch diesen Knick werden die Seitenschenkel 4, 5 aus der Eindringrichtung E zueinander hin abgelenkt. Die V-Kontur 9 des Formstempels 1 begrenzt die Verformung des Zwischenstücks 6 und somit auch den Winkel α, mit dem die Seitenschenkel 4, 5 der Hautklammer 2 aus der Eindringrichtung E abgelenkt werden.

Darüber hinaus wird durch die V-Kontur 9 in Verbindung mit dem Dorn 19, der als Gegenhalter wirkt, die Betätigung des Formstempels 1 blockiert, sobald der Dorn 19 bzw. das darüber liegende Zwischenstück 6 am Scheitelpunkt 21 der V-Kontur 9 des Formstempels 1 anschlägt.

Beim Impulsverfahren (Fall A) wird das Eintreiben der Seitenschenkel 4, 5 und die Verformung 20 in einem Arbeitsgang erledigt. In diesem Zustand ist die Hautklammer 2 bereits in ihrer Endform und Endlage, so dass die Vorrichtung 3 zurückgezogen werden kann.

In Figur 7 ist dementsprechend die geschlossene Wunde 12' mit der Hautklammer 2' in ihrer Endform dargestellt. Durch die Abweichung α von der Eindringrichtung E kann die Hautklammer 2 sich nicht selbsttätig lösen.

Als Sicherung gegen ein derartiges selbsttätiges Lösen können zusätzlich Klebestrips 22 im Bereich der Wunde 12 über den Hautklammern 2 angebracht werden, wie dies anhand Figur 8 erkennbar ist.

Figur 9 veranschaulicht die Zufuhr von Hautklammern aus einem Klammermagazin 23. Eine Vielzahl von Hautklammern wird hierbei über eine Magazinführung 24 abgelegt und, beispielsweise durch Federdruck (nicht näher dargestellt), in Richtung zum Formstempel 1 geschoben. Der Formstempel 1 ist in der Darstellung gemäß Figur 10 eingedrückt, so dass er kurz oberhalb des Dorns 19 steht. Zwischen dem Führungsgehäuse 18 für den Formstempel 1 und dem Dorn 19 ist ein Auslassschlitz 25, durch den die in dieser Darstellung nicht im Einzelnen erkennbaren Hautklammern 2 die Vorrichtung 3 verlassen können.

Eine erfindungsgemäße Vorrichtung 3 gewährleistet durch die gegenüber herkömmlichen Klammerapparaten geringere Ablenkung α der Seitenschenkel 4, 5 der Hautklammer 2 aus der Einbringrichtung, dass Hautquetschungen reduziert wenn nicht ganz vermieden werden und die Hautklammern 2 ohne Spezialgerät herausgezogen werden können.

Darüber hinaus findet die wesentliche Verformung der Hautklammer 2 oberhalb bzw. außerhalb der Haut statt, so dass auch hierdurch die Quetschgefahr reduziert wird. Weiterhin wird durch das Auffahren des Formstempels 1 auf den Dorn 19 gewährleistet, dass der zur Verformung der Klammer 2 erforderliche Druck nicht auf die Haut übertragen wird. Auch hierdurch wird die Quetschgefahr während der Verformung vermindert.

Der Druck zur Verformung der Hautklammer ist völlig unabhängig von dem Druck, mit dem der Operateur die Vorrichtung auf die Haut andrückt. Der Anpressdruck auf der Haut muss letztendes lediglich so hoch sein, dass die Hautklammer 2 in die Haut eindringen kann. Wie beschrieben könnte das Eindringen auch mittels eines Federimpulses erfolgen.

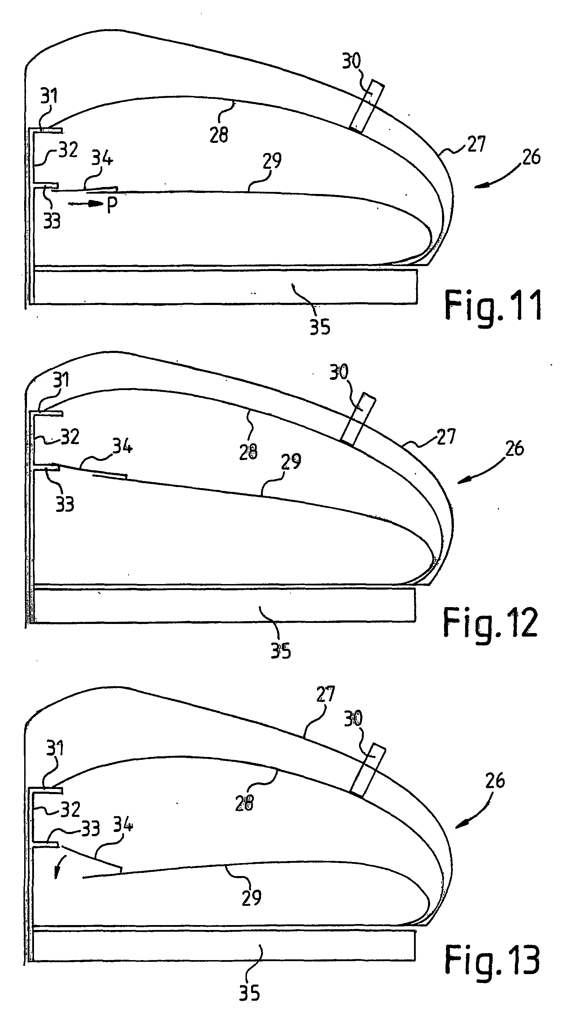

Die Skizze gemäß Figur 11 zeigt eine Vorrichtung 26 mit einem äußeren Rahmen 27, in dem eine Druckfeder 28 sowie eine Hubfeder 29 montiert sind. Eine Stellschraube 30 im Rahmen 27 dient der Verstellung der Vorspannung der Druckfeder 28.

Die Druckfeder 28 ist an einem oberen Quersteg 31 des Formstempels 32 aufgelegt. Ein Rastvorsprung 33 ist zur Verrastung des Auslöseelementes 34 vorgesehen. Das Auslöselement 34 ist beweglich an der Hubfeder 29 befestigt.

Auf der Unterseite der Vorrichtung 26 befindet sich weiterhin ein Klammermagazin 35.

Die skizzierte Vorrichtung 26 arbeitet folgendermaßen. In der in Figur 11 dargestellten Stellung ist die Federkraft der Druckfeder 28 durch die Federkraft der Hubfeder 29 überkompensiert, so dass sich der Formstempel 32 in seiner oberen Position über dem Klammermagazin 35 befindet. Durch Auslösung des Auslöseelementes 34, beispielsweise aufgrund einer Verschiebung in Richtung des Pfeils P wird die Hubfeder 29 vom Formstempel 32 entkoppelt, so dass die Druckfeder 28 in der Lage ist, den Formstempel 32 über den Quersteg 31 nach unten zu drücken. Hierbei wird eine Klammer aus dem Klammermagazin 35 ausgestoßen und in die nicht näher dargestellte, darunter liegende Haut eingebracht.

Nach dem Einbringen der Hautklammer in die Haut liegt die Klammervorrichtung 26 im Zustand gemäß Figur 13 vor. Nunmehr wird durch die Vorspannung der Hubfeder 29 das Auslöseelement 34 wieder in die verrastete Stellung nach unten gebracht. Das Auslöseelement 34 ist hierbei nachgiebig an der Hubfeder 29 befestigt. Diese Funktion könnte jedoch beispielsweise auch durch ein flexibles Auslöseelement 34 bzw. des Rastvorsprungs 33 verwirklicht werden. Nachdem das Auslöseelement 34 sich wieder in der verrasteten Stellung befindet, ist es in der Lage, über die Hubfeder 29 und Rastvorsprung 33 den Formstempel 32 gegen die Kraft der Druckfeder 28 anzuheben, so dass sich wieder die Position gemäß Figur 11 ergibt.

Mit dieser Bauform mit Hilfe von Blattfedern lässt sich eine leichte und einfache Bauweise realisieren, die gut zu reinigen ist. Darüber hinaus ist mit dieser Bauweise ein sterilisierbarer und damit wiederverwendbarer Klammerapparat möglich.

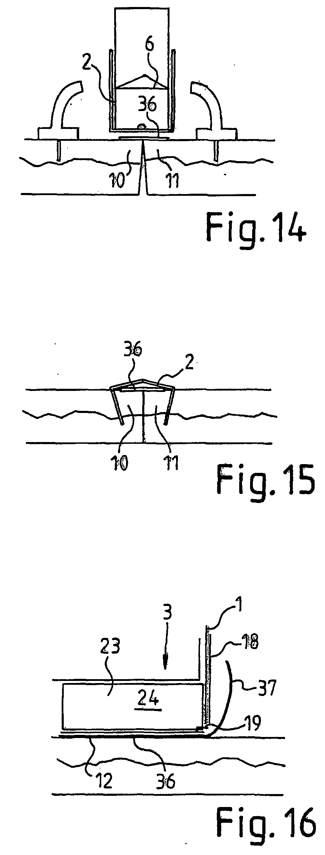

Figur 14 zeigt eine Figur 5 entsprechende Darstellung, wobei nunmehr ein Schutzband 36 unterhalb des Zwischenstücks 6 der Hautklammer 2 über die Wundränder 10, 11 gelegt ist. Nach dem Einbringen der Hautklammer 2 (vgl. Figur 15) ist das Schutzband 36 gemeinsam mit den Wundrändern 10, 11 fixiert.

Figur 16 zeigt eine Figur 9 entsprechend der Darstellung, wobei nunmehr das Schutzband 36 auf der Wunde zwischen der erfindungsgemäßen Vorrichtung 3 und der Wunde 12 eingelegt ist. Das freie Ende 37 des Schutzbands 36 ist aufwärts gebogen und kann beispielsweise in einer nicht näher dargestellten, darüber liegenden Vorratsrolle enden.

Aufgrund der sehr schonenden Klammerung mit einer erfindungsgemäßen Vorrichtung ist der Einsatz dieser Vorrichtung in Anwendungsfällen möglich, wo bislang auf eine Klammerung verzichtet wurde, wie beispielsweise in der Schönheitschirurgie oder bei der Wundversorgung von sichtbaren und anderweitig kosmetisch empfindlichen Arealen, wie z.B. dem Gesicht.

Darüber hinaus ist die erfindungsgemäße Vorrichtung vergleichsweise einfach aufgebaut, so dass problemlos Klammerapparate und Hautklammern in unterschiedlicher Größe bereitgestellt werden können.

### Bezugszeichenliste:

- 1: Formstempel
- 2: Hautklammer
- 3: Vorrichtung
- 4: Seitenschenkel
- 5: Seitenschenkel
- 6: Zwischenstück
- 7: Formzone
- 8: Formzone
- 9: V-Kontur
- 10: Wundrand
- 11: Wundrand
- 12: Wunde
- 13: Lederhaut
- 14: Fettgewebe
- 15: Adaptionszange
- 16: Spitze
- 17: Spitze
- 18: Führungsgehäuse
- 19: Dorn
- 20: Knick
- 21: Scheitelpunkt
- 22: Klebestrip
- 23: Klammermagazin
- 24: Magazinführung
- 25: Auslassschlitz
- 26: Vorrichtung
- 27: Rahmen
- 28: Druckfeder
- 29: Hubfeder
- 30: Stellschraube
- 31: Quersteg
- 32: Formstempel
- 33: Rastvorsprung
- 34: Auslöseelement
- 35: Klammermagazin
- 36: Schutzband
- 37: Ende

## Patentansprüche

1. Hautklammervorrichtung zur Fixierung von wundrändern durch Einbringen und Formen von Hautklammern (2) mit einem Klammerapparat,
- der einen Formstempel (1) sowie ein Magazin (23) für mehrere Hautklammern aufweist, die im Ausgangszustand zwei gerade, zueinander parallele und parallel zu einer geraden Einbringrichtung in das zu klammernde Gewebe stehende Seitenschenkel (4, 5) und ein die Seitenschenkel verbindendes Zwischenstück (6) aufweist,
- wobei Mittel zum Einbringen der senkrechten Seitenschenkel ohne Verformung der Hautklamer bis zum Anschlag auf einen Gegenhalter (19) und zur anschließenden Verformung vorgesehen sind, wobei die Abweichung (α) der Seitenschenkel von der Einbringrichtung (E) im geformten Endzustand kleiner als 45 ° ist und
- eine Druckfeder (28) zum Eindrücken des Formstempels sowie eine Hubfeder (29) als Rückholelement für den Formstempel vorgesehen sind,
**dadurch gekennzeichnet, dass**
- ein Auslöseelement (34) zur Abkopplung der Hubfeder von dem Formstempel und ein Rastelement (33) zum Verrasten der Hubfeder an dem Formstempel nach dem Einbringen der Hautklamer für den Rückholvorgang vorgesehen ist.

2. Vorrichtung nach Anspruch 1, **dadurch gekennzeichnet, dass** eine Verformung des Zwischenstücks (6) vorgesehen ist.

3. Vorrichtung nach einem der vorgenannten Ansprüche, **dadurch gekennzeichnet, dass** zwei Formzonen (7, 8) am Formstempel (1) vorgesehen sind.

4. Vorrichtung nach einem der vorgenannten Ansprüche, **dadurch gekennzeichnet, dass** der Gegenhalter (19) als Dorn ausgebildet ist.

5. Vorrichtung nach einem der vorgenannten Ansprüche, **dadurch gekennzeichnet, dass** der Formstempel (1) eine V-förmige Kontur (9) aufweist.

6. Vorrichtung nach einem der vorgenannten Ansprüche, **dadurch gekennzeichnet, dass** die Hautklammer (2) im Ausgangszustand ein gerades und im geformten zustand ein geknicktes Zwischenstück (6) aufweist.

7. Vorrichtung nach einem der vorgenannten Ansprüche, **dadurch gekennzeichnet, dass** ein Magazin (23) für mehrere Hautklammern (2) vorgesehen ist.

8. Vorrichtung nach einem der vorgenannten Ansprüche, **dadurch gekennzeichnet, dass** die Abweichung α der Seitenschenkel (4, 5) von der Einbringrichtung E ungefähr einem Winkel von 30° entspricht.

9. Vorrichtung nach einem der vorgenannten Ansprüche, **dadurch gekennzeichnet, dass** das Auslöseelement (34) zugleich ein Rastelement ist.

10. Vorrichtung nach einem der vorgenannten Ansprüche, **dadurch gekennzeichnet, dass** die Druck- und/oder Hubfeder (28, 29) als Blattfeder ausgebildet ist.

11. Vorrichtung nach einem der vorgenannten Ansprüche, **dadurch gekennzeichnet, dass** ein Stellelement (30) zur Einstellung der Vorspannung der Druck- und/oder Hubfeder (28, 29) vorgesehen ist.

12. Vorrichtung nach einem der vorgenannten Ansprüche, **dadurch gekennzeichnet, dass** ein Schutzband (36) vorgesehen ist, das zwischen Haut und Hautklammer anzuordnen ist.

13. Vorrichtung nach einem der vorgenannten Ansprüche, **dadurch gekennzeichnet, dass** ein Vorratsmagazin für das Schutzband (36) vorgesehen ist.

## Claims

1. Skin clip device for fixing the edges of wounds by introducing and forming skin clips (2) with a clip apparatus, comprising a forming die (1) and a magazine (23) for a plurality of skin clips which in the original state comprise two straight side legs (4, 5) located parallel to one another and parallel to a straight direction of introduction into the tissue to be clipped and comprising an intermediate piece (6) connecting the side legs, means for introducing the vertical side legs, without deformation of the skin clips, until they abut a counter holder (19) and for subsequent deformation being provided, the deviation (α) of the side legs from the direction of introduction (E) being less than 45° in the formed end state, and a compression spring (28) for pressing in the forming die and a lift spring (29) as a return element for the forming die being provided, **characterised in that** a release element (34) for uncoupling the lifting spring from the forming die and a latching element (33) for latching the lifting spring to the forming die after introduction of the skin clips are provided for the return operation.

2. Device according to claim 1, **characterised in that** deformation of the intermediate piece (6) is provided.

3. Device according to any of the preceding claims, **characterised in that** two forming zones (7, 8) are provided on the forming die (1).

4. Device according to any of the preceding claims, **characterised in that** the counter holder (19) is constructed as a mandrel.

5. Device according to any of the preceding claims, **characterised in that** the forming die (1) has a V-shaped contour (9).

6. Device according to any of the preceding claims, **characterised in that**, in the original state, the skin clip (2) has a straight intermediate piece (6) and in the formed state a bent intermediate piece (6).

7. Device according to any of the preceding claims, **characterised in that** a magazine (23) for a plurality of skin clips (2) is provided.

8. Device according to any of the preceding claims, **characterised in that** the deviation α of the side legs (4, 5) from the direction of introduction E corresponds approximately to an angle of 30°.

9. Device according to any of the preceding claims, **characterised in that** the release element (34) is simultaneously a latching element.

10. Device according to any of the preceding claims, **characterised in that** the compression and/or lifting spring (28, 29) is constructed as a leaf spring.

11. Device according to any of the preceding claims, **characterised in that** an adjusting element (30) for adjusting the bias of the compression and/or lifting spring (28, 29) is provided.

12. Device according to any of the preceding claims, **characterised in that** a protective strip (36) is provided which is to be arranged between skin and skin clip.

13. Device according to any of the preceding claims, **characterised in that** a supply magazine is provided for the protective strip (36).

## Revendications

1. Dispositif d'agrafage de peau pour fixer les bords d'une plaie par la pose et le formage d'agrafes de peau (2) avec un appareil d'agrafage
- qui comprend un poinçon de formage (1) ainsi qu'un magasin (23) pour plusieurs agrafes, lesquelles présentent dans l'état initial deux branches latérales (4, 5) droites, parallèles entre elles et parallèles à une direction de pose rectiligne dans le tissu à agrafer, et une partie intermédiaire (6) reliant les branches latérales,
- des moyens étant prévus pour la pose des branches latérales verticales sans formage de l'agrafe de peau jusqu'à la butée contre un contre-support (19) et pour le formage ayant lieu par la suite, l'écart (α) des branches latérales par rapport à la direction de pose (E) dans l'état final avec formage étant inférieur à 45° et
- un ressort de pression (28) qui sert à enfoncer le poinçon de formage ainsi qu'un ressort de levage (29) qui sert d'élément de rappel du poinçon de formage étant prévus,
**caractérisé en ce qu'**il est prévu
- un élément de déclenchement (34) pour désaccoupler le ressort de levage du poinçon de formage et un élément d'encliquetage (33) pour encliqueter le ressort de levage sur le poinçon de formage après la pose de l'agrafe de peau pour le processus de rappel.

2. Dispositif selon la revendication 1,
**caractérisé en ce qu'**il est prévu un formage de la partie intermédiaire (6).

3. Dispositif selon l'une quelconque des revendications précédentes,
**caractérisé en ce qu'**il est prévu deux zones de formage (7, 8) sur le poinçon de formage (1).

4. Dispositif selon l'une quelconque des revendications précédentes,
**caractérisé en ce que** le contre-support (19) est conçu comme un mandrin.

5. Dispositif selon l'une quelconque des revendications précédentes,
**caractérisé en ce que** le poinçon de formage (1) présente un contour en forme de V (9).

6. Dispositif selon l'une quelconque des revendications précédentes,
**caractérisé en ce que** l'agrafe de peau (2) présente une partie intermédiaire (6) droite dans l'état initial et coudée dans l'état formé.

7. Dispositif selon l'une quelconque des revendications précédentes,
**caractérisé en ce qu'**il est prévu un magasin (23) pour plusieurs agrafes (2).

8. Dispositif selon l'une quelconque des revendications précédentes,
**caractérisé en ce que** l'écart α des branches latérales (4, 5) par rapport à la direction de pose E correspond à un angle d'environ 30°.

9. Dispositif selon l'une quelconque des revendications précédentes,
**caractérisé en ce que** l'élément de (34) est en même temps un élément d'encliquetage.

10. Dispositif selon une quelconque des revendications précédentes,
**caractérisé en ce que** le ressort de pression et/ou de levage (28, 29) est conçu sous forme de lame.

11. Dispositif selon l'une quelconque des revendications précédentes,
**caractérisé en ce qu'**il est prévu un élément de réglage (30) pour régler la tension initiale du ressort de pression et/ou du ressort de levage (28, 29).

12. Dispositif selon l'une quelconque des revendications précédentes,
**caractérisé en ce qu'**il est prévu une bande de protection (36) qui est à disposer entre la peau et l'agrafe.

13. Dispositif selon l'une quelconque des revendications précédentes,
**caractérisé en ce qu'**il est prévu un magasin de réserve de bande de protection (36).
